# EUROPEAN PATENT APPLICATION

(11) **EP 4 428 247 A1**
(43) Date of publication of application: **11.09.2024**
(21) Application number: 22890441.3
(22) Date of filing: 04.11.2022
(51) Int. Cl.: C12Q 1/6844, C12Q 1/686

(54) **METHOD OF AMPLIFICATION OR DETECTION OF TARGET MATERIAL USING NANOPARTICLES**

(30) Priority: 05.11.2021 KR 20210151779; 18.11.2021 KR 20210159555
(71) Applicant: Seoul National University R&DB Foundation, Seoul 08826 (KR)
(72) Inventor: PARK, Eunhye, Seoul 04052 (KR); NAM, Jwa Min, Seoul 04773 (KR); KIM, Sungi, Seoul 08098 (KR)
(74) Representative: Elkington and Fife LLP
(86) International application number: PCT/KR2022/017238
(87) International publication number: WO 2023/080710

(57) **Abstract**

The present application provides nanoparticles comprising a plurality of partially double-stranded probes, and a composition, array, kit, apparatus, and/or method using the nanoparticles for amplification, detection, quantification, identification, and/or visualization of a target material. The nanoparticles may each comprise (1) a template strand which comprises (i) a target-cloning site comprising a complementary sequence to a target oligonucleotide and (ii) a target-binding site comprising a complementary sequence to a portion of a target nucleic acid fragment, and (2) a target-cloning strand which comprises the same sequence as the target oligonucleotide and hybridizes to the target-cloning site of the template strand.

## Description

### Technical Field

### CROSS-REFERENCE TO RELATED APPLICATIONS

This patent application claims priority to and the benefit of Korean Patent Application Nos. 10-2021-0151779 and 10-2021-0159555 filed with the Korean Intellectual Property Office on November 05, 2021 and November 18, 2021, respectively, the disclosures of which are incorporated herein by reference.

Provided herein are a nanoparticle including a plurality of partially double-stranded probes thereon, and a composition, an array, a kit, a device, and/or a method for amplifying, detecting, quantifying, identifying, and/or visualizing a target material, using same.

### Background Art

Onsite sensing and diagnostic methods, particularly on a portable platform, are highly beneficial in that they offer rapid, inexpensive and straightforward means to identify the sources for acute diseases, infections or biological and chemical warfare agents with no or minimal instrumentation. Among these methods, colorimetric methods can be powerful and widely applicable since they can offer naked-eye detection of targets without a need for any expertise or instrumentation. However, in most cases, nanoparticle-based colorimetric assays rely only on the optical properties of nanomaterial, and it limits sensitivity, reliability and quantification capability. Therefore, colorimetric assays are typically unreliable with a narrow range of practical use, and have not been well explored for high-sensitivity-required applications. Ultrasensitive detection methods are typically based on polymerase chain reaction (PCR) or highly sophisticated amplification, sensing and imaging techniques.

Therefore, there is a need for the development of a more convenient and sensitive biological detection technology.

### Disclosure

### Technical Problem

To offer sensitive, rapid, inexpensive, and straightforward means for detecting a target material, the present disclosure provides a nanoparticle having a plurality of partially double-stranded probes, and an array, a kit, a device, and/or a method for amplifying, detecting, quantifying, identifying, and/or visualizing a target material, using same.

More specifically, an aspect provides a partially double-stranded oligonucleotide including:
(1) a first strand (template strand) including:
   (i) a target-cloning site hybridizable with a target oligonucleotide, and
   (ii) a target-binding site hybridizable with or complementary to a part of the target oligonucleotide; and
(2) a second strand (target-cloning strand or target amplifier strand) including the same sequence as the target oligonucleotide.

Another aspect provides a nanoparticle including two or more partially double-stranded oligonucleotides on the surface thereof, each partially double-stranded oligonucleotide being as defined above.

A further aspect provides a composition, an array, a kit, and/or a device for amplification, detection, quantification, identification, and/or visualization of a target material, each including: (a) a substrate particle including a target-capturing strand partially hybridizable with a target oligonucleotide on the surface thereof; and/or the nanoparticle.

A yet further aspect provides a method for amplification, detection, quantification, identification, and/or visualization of a target material, the method using: (a) a substrate particle including a target-capturing strand partially hybridizable with a target oligonucleotide on the surface thereof; and/or (b) the nanoparticle.

Yet another aspect provides a use of (a) a substrate particle including a target-capturing strand partially hybridizable with a target oligonucleotide on the surface thereof; and/or (b) the nanoparticle for amplification, detection, quantification, identification, and/or visualization of a target material.

### Solution to the Problem

Provided herein are a nanoparticle with a specific structure and a "nanoparticle chain reaction" (NCR) technology utilizing same. This NCR technology, which is a nucleic acid molecule amplification method, distinguishes itself from conventional polymerase chain reaction (PCR) with respect to the enzyme-free method that uses specially structured nanoparticles as an alternative to polymerases. With more simplicity and significantly higher amplification efficiency compared to PCR, the NCR technology can find advantageous applications in the detection of nucleic acids and various target materials which can be amplified through nucleic acid molecules.

Below, a detailed description will be given of the present disclosure:

### Definition of Terms

As used herein, the term "target material" refers to a material to be amplified, detected, quantified, identified, and/or visualized finally in a composition, array, kit, device, and/or method for amplification, detection, quantification, identification, and/or visualization provided herein and is intended to encompass all bioactive substances. In an embodiment, the target material may be at least one selected from the group consisting of organisms (various eukaryotes or prokaryotes; for example, viruses, bacteria, fungi, etc.) or bioactive substances, e.g., nucleic molecules (e.g., full-length genomes (DNA or RNA), genomic segment, genes, genetic fragments, siRNA, miRNA, biomarker, and other DNA or RNA fragments), proteins, peptides, polymers, small molecule chemicals (e.g., chemical drugs including at least one selected from the group consisting of various metal ions and organic compounds), but with no limitations thereto. In an embodiment, the bioactive substances such as nucleic acid molecules, proteins, peptides, etc. may be derived from the organisms described above (e.g., viruses, bacteria, fungi, etc.), but are not limited thereto, and may be those recombinantly or chemically synthesized.

As used herein, the term "sample" refers to any biological and/or environmental sample intended for the detection and/or measurement of the presence or level of a target material. For example, biological samples can be selected from a group consisting of cells, tissues, blood, lymph, saliva, sputa, nasal secretions, urine, feces, and other bodily fluids isolated from a subject (patient), but are not limited thereto. The subjects may include humans, livestock (e.g., cows, pigs, horses, sheep, goats, dogs, cats, etc.), birds (chickens, ducks, geese, turkeys, ostriches, quails, etc.), but are not limited thereto.

As used herein, the term "target oligonucleotide" refers to an oligonucleotide that is directly amplified by the chain reaction provided herein and is also referred to as a "target strand". The two terms are interchangeable each other for the equivalent meanings. For instance, it is understood that:
- when the target material is a nucleic acid molecule, its entirety (in case that the target material is a nucleic acid molecule, such as a DNA fragment and/or RNA fragment) or part (an amplification target sequence, e.g., a unique part of the nucleic acid molecule) is indicated by the term target oligonucleotide; and
- when the target material is a protein, a peptide, a polymer, or a small molecule chemical, the term target oligonucleotide is same as the target-cloning site described below.

The term "template strand", as used herein, refers to a single-stranded oligonucleotide used as a template in the NCR technology provided herein for the amplification of a target oligonucleotide and is intended to encompass a target-cloning site with a nucleic acid sequence complementary to the full-length sequence of a target oligonucleotide and a target-binding site with a nucleic acid sequence complementary to part of a target oligonucleotide. The template strand may be an oligonucleotide with a length of 10-1000 nt, 10-500 nt, 10-200 nt, 10-100 nt, 10-90 nt, 10-80 nt, 10-70 nt, 10-60 nt, 10-55 nt, 10-50 nt, or 10-45 nt, but is not limited thereto.

As used herein, the term "target-cloning strand" refers to a single-stranded oligonucleotide that has the same nucleic acid sequence as a target oligonucleotide or a partial sequence to be amplified in the target and can hybridize (complementarily bind) with the target-cloning site of the template strand.

As used herein, the term "target-capturing strand" denotes a single-stranded oligonucleotide that functions to capture a target oligonucleotide onto a substrate particle, having a nucleic acid sequence complementary to a portion different from the complementary portion to the target-binding site of the template strand in the target oligonucleotide.

The expression "a nucleic acid molecule or protein having a specific nucleic acid sequence or amino acid sequence" as used herein, means that the nucleic acid molecule or protein includes or consists essentially of the sequence

### Partially double-stranded oligonucleotide

An embodiment provides a partially double-stranded oligonucleotide including:
(1) a first strand (long (extended) strand; hereinafter referred also to as "template strand") including:
   (i) a target-cloning site hybridizable with (complementary to) a target oligonucleotide, and
   (ii) a target-binding site hybridizable with or complementary to a portion of the target oligonucleotide, wherein the target-cloning site and the target-binding site are linked to each other (e.g., the 3'-end of the target-cloning site is bonded to the 5'-end of the target-binding site, or the 5'-end of the target-cloning site is linked to the 3'-end of the target-binding site); and
(2) a second strand (short strand (shorter than the first strand); hereafter referred also to as target-cloning strand or target amplifier strand, the two terms being interchangeable each other for the equivalent meaning) including the same sequence as the target oligonucleotide (that is, including a sequence complementary to (hybridizable with) a target-cloning site of the first strand),
   wherein the target-cloning strand hybridizes with (complementarily binds to) the target-cloning site of the template strand to form a double-stranded structure (that is, a double-stranded portion (site) of the partially double-stranded oligonucleotide), with the target-binding site of the template strand existing in a single-stranded form.

The template strand may be an oligonucleotide with a length of 10-1000 nt, 10-500 nt, 10-200 nt, 10-100 nt, 10-90 nt, 10-80 nt, 10-70 nt, 10-60 nt, 10-55 nt, 10-50 nt, or 10-45 nt, but is not limited thereto. The target-cloning strand may be an oligonucleotide that is so long as to hybridize with the target-cloning site of the first strand with the target-binding site remaining exposed. For example, the target-cloning strand may be identical to a target oligonucleotide.

### Nanoparticles (NCR probe)

Another embodiment provides a nanoparticle having one or more (e.g., two or more) partially double-stranded oligonucleotides conjugated to the surface thereof. The partially double-stranded oligonucleotide is as described in the foregoing. For instance, the partially double-stranded oligonucleotide may be immobilized (attached) to the surface of the nanoparticle directly or through a linker (first linker) from one end of the template strand (e.g., in both ends of the target-cloning site in the template strand, one end other than the opposite end linked to the target-binding site).

In other words, the nanoparticle may include, on the surface thereof,
(1) a template strand including: (i) a target-cloning site immobilized directly or through a linker (first linker) to the surface and (ii) a target-binding site linked to one end of the target-cloning site (i.e., an end different from the end immobilized to the surface of the nanoparticle); and
(2) a target-cloning strand hybridizable with (complementary to) the target-cloning site of the template strand. In this regard, the target-cloning site of the template strand (first strand) hybridizes with the target-cloning strand (second strand) to form a partially double-stranded oligonucleotide, with an overhang of the target-binding site in the template strand.

One or more, 2 or more, 10 or more, 20 or more, 50 or more, 70 or more, 100 or more, 120 or more, 150 or more, or 170 or more of the partially double-stranded oligonucleotides are immobilized (attached) to the surface of one nanoparticle (limitless upper values; as many partially double-stranded oligonucleotides as possible may be attached to the nanoparticle surface (appropriately determined based on the nanoparticle surface area), e.g., 10,000, 5,000, 2,500, 1,000, 750, 500, 250, or 200 partially double-stranded oligonucleotides may be employed), but with no limitations thereto.

In an embodiment, the nanoparticles may be made of a material capable of visualization (e.g., naked-eye-detectable) and/or conjugation to biomolecules (e.g., nucleic acid molecules, antibodies, etc.), for example, metal, silica, hydrogel, carbon nanotubes, quantum dots, such as gold (e.g., gold nanoparticles), silver (e.g., silver nanoparticles), platinum, copper, an alloy (e.g., two or more selected from gold, silver, platinum, coppers, and so on), but with no limitations thereto. In another embodiment, the nanoparticles may be visualized by a fluorescent label contained in the oligonucleotides conjugated to the nanoparticles. The nanoparticles may have a size (average diameter) of 1 to 1000nm, 1 to 500nm, 1 to 200nm, 1 to 100nm, 10 to 1000nm, 10 to 500nm, 10 to 200nm, or 10 to 100nm, but with no limitations thereto. No particular limitations are imparted to the structure and/or morphology of the nanoparticles. By way of example, the nanoparticles may be in a solid structure, a core-shell structure, or a hollow structure, and/or have a spherical, oval, tubular, or polygonal shape, with no limitations thereto. Depending on the type of nanoparticles, the levels of biomolecule conjugation, amplification, and/or visibility can be controlled.

In an embodiment, the nanoparticles may be used as probes (NCR probes) for detecting or amplifying a target oligonucleotide.

As described in the foregoing, the partially double-stranded oligonucleotides or the nanoparticles having the partially double-stranded oligonucleotides attached to on the surface thereof include a region (target-binding site) hybridizable with (having a complementary sequence to) a target oligonucleotide, and a target-cloning strand having the same nucleic acid sequence as the target oligonucleotide. Thus, the partially double-stranded oligonucleotides or the nanoparticles can be advantageously used for the detection and/or amplification of the target oligonucleotide or target material.

In an embodiment, if the target material is a material other than a nucleic acid molecule, the nanoparticles may further include a binding material for the target material in addition to the aforementioned partially double-stranded oligonucleotides. The binding material for the target material may be at least one selected from a group consisting of antibodies, aptamers, small molecular chemicals, etc., that can bind (e.g., specifically bind) to a part of the target material.

### Substrate particles

As used herein, term "substrate particle" refers to any form of particles that have a surface capable of immobilizing capturing materials for the target material (e.g., oligonucleotides, antibodies, peptides, polymer compounds, etc.).

Provided according to an embodiment is a substrate particle that has one or more (e.g., two or more) target-capturing strands (or target capturing oligonucleotides) immobilized (attached, conjugated) to the surface thereof, wherein the target-capturing strands each are hybridizable with or have a complementary sequence to a part of the target oligonucleotide. These target-capturing strands may be immobilized (attached) to the surface of the substrate particles directly or via a linker (second linker).

As described later, since as many nanoparticles as the number obtained by multiplying the number of target-cloning strand contained in the nanoparticles by the number of NCR cycles are bonded to the substrate particles, the number of target-capturing strands immobilized (attached) to the surface of each substrate particle should be sufficiently large to cover the number of nanoparticles. For example, the number of target-capturing strands per substrate particle may be 10 or more, 50 or more, 10² or more, 10³ or more, 10⁵ or more, 10⁶ or more, 10⁷ or more, or 10⁸ or more (limitless upper values: as many partially oligonucleotides as possible may be attached to the substrate particle surface (appropriately determined based on the substrate particle surface area), e.g., 10¹², 10¹¹, 10¹⁰, or 10⁹), but with no limitations thereto

The substrate particles may be made of solid materials and may include, but are not limited to, magnetic particles or beads (e.g., magnetic beads) to facilitate the collection/purification of the composite formed with the aforementioned nanoparticles. For example, the substrate particles can be composed of metals such as gold (e.g., gold nanoparticles), silver (e.g., silver nanoparticles), platinum, copper, alloys (including two or more selected from the group consisting of gold, silver, platinum, copper, etc.), as well as materials like silica, hydrogels, carbon nanotubes, quantum dots, but are not limited thereto. Additionally, the size (average diameter) of the substrate particles should be sufficiently larger than the nanoparticles and may be at least twice, five times, ten times, twenty times, thirty times, forty times, or fifty times larger than the size (average diameter) of the nanoparticles, for example, ranging from 0.1 to 100 µm, 0.1 to 50 µm, 0.1 to 20 µm, 0.1 to 10 µm, 0.1 to 8 µm, 0.1 to 5 µm, 0.1 to 3 µm, 0.5 to 100 µm, 0.5 to 50 µm, 0.5 to 20 µm, 0.5 to 10 µm, 0.5 to 8 µm, 0.5 to 5 µm, 0.5 to 3 µm, 1 to 100 µm, 1 to 50 µm, 1 to 20 µm, 1 to 10 µm, 1 to 8 µm, 1 to 5 µm, or 1 to 3 µm, but with no limitations thereto. No particular limitations are imparted to the structure and/or morphology of the substrate particles. By way of example, the substrate particles may be in a solid structure, a core-shell structure, or a hollow structure, and/or have a spherical, oval, tubular, or polygonal shape, with no limitations thereto.

In an embodiment, if the target material is a material other than a nucleic acid molecule, the substrate particles may further include a capturing material for the target material in addition to the aforementioned target-capturing strand. The capturing material for the target material may be at least one selected from a group consisting of antibodies, aptamers, small molecular chemicals, etc., that can bind (e.g., specifically bind) to a part of the target material. In this regard, the capturing material for the target material included in the substrate particles and the binding material for the target material included in the nanoparticles may bind to different parts of the target material. That is, the capturing material and the binding material do not overlap for the binding sites on the target material.

In this application, a linker (first linker) available to immobilize (attach) the template strand (target-cloning site) to the surface of nanoparticles, and a linker (second linker) available to immobilize (attach) the target-capturing strand to the surface of substrate particles may be appropriately selected from all materials capable of attaching oligonucleotides to the surface of solid particles. For example, the linkers may be selected from the group consisting of chemical functional groups (e.g., thiol (SH), amine (NH₂)) and binding proteins (e.g., biotin), but are not limited thereto, and can be appropriately selected based on the surface material of the attachment particles. The linkers may be bound to the terminals at which the template strand and target-capturing strand respectively bind to the surface of nanoparticles and substrate particles (e.g., 3' terminus for the template strand and 5' terminus for the target-capturing strand) and/or may be attached to the surface of nanoparticles and/or substrate particles. Additionally, the template strand and/or target-capturing strand may include a spacer at their terminals binding respectively to the nanoparticle and substrate particle surfaces (e.g., 3' terminus for the template strand, 5' terminus for the target-capturing strand) to maintain a predetermined distance from the surface of nanoparticles and/or substrate particles (if the strands include linkers, the spacers can be included between the template strand and the first linker (first spacer), and/or between the target-capturing strand and the second linker (second spacer)). The spacer may be appropriately selected from all materials capable of providing a predetermined length/volume among those able to provide a certain distance, and can be one or more selected from a group consisting of oligonucleotides (e.g., Poly A (e.g., A2~20)), polymers (e.g., polyethylene glycol (PEG)), alkyls of 1-6 carbon atoms, but are not limited thereto. For example, the spacer may be poly A, PEG, or a combination thereof (first spacer), or poly A, alkyls of 1-6 carbon atoms, or a combination thereof (second spacer), but is not limited to these.

### Detection and/or amplification of target material (nucleic molecule)

Another embodiment provides a composition, an array, a kit, and/or a device including one or more substrate and one or more nanoparticles described above for the amplification, detection, quantification, identification, and/or visualization of a target material. The composition, array, kit, and/or device may include the substrate particles and/or nanoparticles dispersed and/or suspended in an appropriate medium (e.g., liquid medium).

In detail, the composition, array, kit, and/or device may include:
(a) at least one (e.g., two or more) substrate particle having at least one (e.g., two or more) target-capturing strand (or target capturing oligonucleotide) immobilized (grafted or bonded) to the surface thereof, the target-capturing strand including a sequence hybridizable with or complementary to a part of a target oligonucleotide; and
(b) at least one (e.g., two or more) nanoparticle including at least one (e.g., two or more) partially double-stranded oligonucleotide on the surface thereof. The nanoparticle and the partially double-stranded oligonucleotide are as described above.

In an embodiment, the composition, array, kit, and/or device for the amplification, detection, quantification, and/or visualization of a target material may include:
(a) a substrate particle having at least one (e.g., two or more) target-capturing strand immobilized (conjugated or bonded) to the surface thereof, the target-capturing strand including a sequence hybridizable with or complementary to a part (first part) of a target oligonucleotide; and
(b) at least one (e.g., two or more) nanoparticle having at least one (e.g., two or more) partially double-stranded oligonucleotide on the surface thereof, the partially double-stranded oligonucleotide including:
   (1) a first strand (template strand) including
      (i) a target-cloning site hybridizable with (complementary to) the target oligonucleotide, and
      (ii) a target-binding site hybridizable with (complementary to) a part (second part) of the target oligonucleotide, wherein the target-cloning site and the target-binding site are linked to each other (for example, the 3'-end of the target-cloning site may be bonded to and the 5'-end of the target-binding site or the 5'-end of the target-cloning site may be linked to the 3'-end of the target-binding site); and
   (2) a second strand (target-cloning strand) including the same sequence as the target oligonucleotide (that is, sequence complementary to (hybridizable with) the target-cloning site of the template strand,
wherein the target-cloning strand hybridizes with the target-cloning site of the template strand to form a double-stranded structure (i.e., double-stranded part (site) of the, partially double-stranded oligonucleotide, with an overhang of the target-binding site in the template strand.

The target-capturing strand consists essentially of a sequence hybridizable with a part (first part) of the target oligonucleotide, with one end (e.g., 5'- or 3'-end) thereof immobilized (conjugated) to the surface of the substrate particle directly or via a linker (second linker).

In the target oligonucleotide, the part (first part) with which the target-capturing strand on the substrate particle hybridizes and the part (second part) with which the target-binding site of the first strand on the nanoparticle hybridizes may be designed not to overlap each other. For example, in the case where the target-capturing strand hybridizes with a 5'-end part (first part) of the target oligonucleotide, the target-binding site may be designed to hybridize with the 3'-end part (second part) of the target oligonucleotide. In another example, the target-capturing strand may hybridize with a 3'-end part (first part) of the target oligonucleotide while the target-binding site hybridizes with a 5'-end part (second part) of the target oligonucleotide.

In the composition, array, kit, and/or device provided in this application, when the target oligonucleotide (single-stranded) or a sample containing same comes into contact with the substrate particles, a part (first part) of the target oligonucleotide hybridizes with one or more target-capturing strands on the substrate particles while at least one second part of the target oligonucleotide remains single-stranded. The at least one single-stranded part then binds to the target-binding site within the template strand on the nanoparticles whereby at least one nanoparticle binds to the substrate particle to form a substrate particle-nanoparticle sandwich complex. Subsequently, through dehybridization treatment, the target-cloning strand in each nanoparticle is dehybridized and released from the target-cloning site of the template strand. Since the released target-cloning strand has the same nucleic acid sequence as the target oligonucleotide, the released target-cloning strand can re-hybridize with the first part of the target-capturing strand on the substrate particles, thus repeating subsequent cycles. Through this process, as cycles repeat, the number of nanoparticles bound and accumulated (concentrated) on the surface of the substrate particles increases exponentially. Theoretically, based on one substrate particle, the number of nanoparticles that bind and accumulate on the substrate particle is equal to the number of target-cloning strands in the nanoparticles multiplied by the number of cycles [(number of target-cloning strands per nanoparticle)ⁿ, where n is the number of cycles]. This results in the amplification of the target oligonucleotide and/or enrichment of nanoparticles to a detectable level, such as visually detectable levels (e.g., detection of the presence and/or concentration of the target oligonucleotide is facilitated by the color change of the solution due to the accumulated nanoparticles).

Another embodiment provides a method for amplification, detection, quantification, identification, and/or visualization of a target material using the aforementioned substrate particles and nanoparticles. The method may include the steps of
(A) adding (or applying or contacting) a target oligonucleotide or sample to a substrate particle having a plurality of target-capturing strands on the surface thereof to hybridize a part (first part) of the target oligonucleotide with the target-capturing strands;
(B) adding (or applying or contacting) a plurality of nanoparticles to the mixture obtained in step (A) to form complexes of the substrate particles and nanoparticles, where each nanoparticle includes a plurality of partially double-stranded oligonucleotides on the surface thereof, each partially double-stranded oligonucleotide including:
   (1) a first strand (template strand) including:
      (i) a target-cloning site hybridizable with (complementary to) the target oligonucleotide, and
      (ii) a target-binding site hybridizable with or complementary to a part (second part) of the target oligonucleotide; and
   (2) a second strand (target-cloning strand) including the same sequence as the target oligonucleotide,
      wherein complexes of the substrate particles and nanoparticles are formed through the hybridization of the part (second part) of the target oligonucleotide, which is partially hybridized with the target-capturing strands on the surface of the substrate particles, with the target-binding site; and
(C) dehybridizing the partially double-stranded oligonucleotide and/or disassembling the complexes to release the target-cloning strand that has the same nucleic acid sequence as the target oligonucleotide.

In step (A), when the target oligonucleotide or sample is added to the substrate particles that include a plurality of target-capturing strands on the surface thereof, the target-capturing strands can hybridize with a part (first part) of the target oligonucleotide, thereby capturing the target oligonucleotide present within the sample.

The plurality of nanoparticles used in step (B) each include a plurality of partially double-stranded oligonucleotides on the surface thereof, each of the partially double-stranded oligonucleotides including:
(1) a first strand (template strand) including:
   (i) a target-cloning site hybridizable with (complementary to) the target oligonucleotide, and
   (ii) a target-binding site hybridizable with or complementary to a part (second part) of the target oligonucleotide, wherein the target-cloning site and target-binding site are linked to each other (for example, the 3'-terminus of the target-cloning site may be bonded to the 5'-terminus of the target-binding site, or the 5'-terminus of the target-cloning site may be connected to the 3'-terminus of the target-binding site); and
(2) a second strand (target-cloning strand) including the same sequence as the target oligonucleotide (i.e., includes a sequence complementary to (hybridizing with) the target-cloning site of the first strand).

In this regard, the target-cloning strand hybridizes with the target-cloning site of the template strand to form a double-stranded structure (i.e., the double-stranded part of the partially double-stranded oligonucleotide), and the target-binding site of the template strand remains in a single-stranded form and thus can potentially hybridize with another part (second part) of the target oligonucleotide, which is partially hybridized with the target-capturing strands on the substrate particle, thereby allowing for the formation of a sandwich complex assembly between the substrate particles and nanoparticles. In essence, the first part of the target oligonucleotide binds to (hybridizes with) the target-binding site on the nanoparticles' template strand, while the second part (not overlapping with the first part) binds to (hybridizes with) the target-capturing strand on the substrate particles.

In step (C), the dehybridization of the partially double-stranded oligonucleotide and/or the disassembly of the sandwich complex assembly (e.g., through dehybridization) can be performed by conventional means of dehybridization. For example, the dehybridization may be carried out by one or more selected from a group consisting of an increase in pH (e.g., under basic conditions), an increase in temperature (e.g., increasing by 50 to 100°C, 52 to 100°C, 55 to 100°C, 50 to 90°C, 52 to 90°C, 55 to 90°C, etc.), and a decrease in salt concentration, compared to the hybridization reaction. In an embodiment (an Example of this application), the dehybridization was performed by replacing the salt solution with distilled water and lowering the temperature, but with no limitations thereto. Through this process, the target oligonucleotide hybridized with the target-capturing strand and target-binding site, and/or the target-cloning strand hybridized with the target-cloning site (the same nucleic acid therebetween) are each dehybridized and released in single-stranded form. Moreover, during this process, the binding (hybridization) of the target oligonucleotide to both the target-binding site of the template strand on the nanoparticle and the target-capturing strand on the substrate particle is also disassembled through dehybridization, separating (releasing) the nanoparticles and substrate particles that were forming complexes through the target oligonucleotide. The released target-cloning strand and nanoparticles can be used in subsequent cycles.

The method may further include, after step (B), an step of separating, collecting, and/or concentrating the complexes (B-1), wherein step (C) can be performed on the separated, collected, and/or concentrated complexes. The separation, collection, and/or concentration of the complexes can be performed by methods such as applying a magnetic field (e.g., using magnets; in case the substrate particles are magnetic) and/or centrifugation, but with no limitations thereto. The method chosen can be appropriately selected based on the material and characteristics of the used particles, among other factors.

In an embodiment, steps (A) to (C) may be repeated more than twice (where steps (A) through (C) constitute one cycle). In this case, the target-cloning strand released in single-stranded form in step (C), which has the same sequence as the target oligonucleotide, is used again as the target oligonucleotide in step (A), and the nanoparticles released in step (C) are used as NCR probes for the next cycle. Thus, one target oligonucleotide is amplified as many as the number of target-cloning strands contained in one nanoparticle, and in the next cycle, the number of nanoparticles equal to the number of target-cloning strands binds to one substrate particle as the steps proceed. Therefore, one target oligonucleotide is amplified as many times as the number of target-cloning strands included in the nanoparticles multiplied by the number of cycles. That is, the number of target oligonucleotides following the cycle execution can be mⁿ times the initial number (where m is the number of target-cloning strands per nanoparticle (174 is exemplified in the embodiment), and n is the number of cycles). Compared to the amplification level of conventional PCR, which is 2ⁿ times the initial number, the amplification level of the NCR technology provided herein is significantly higher.

In an embodiment, the number of target-cloning strands per nanoparticle (m) may be 2 or more, 10 or more, 20 or more, 50 or more, 70 or more, 100 or more, 120 or more, 150 or more, or 170 or more (limitless upper value: as many oligonucleotides as possible may be attached to the nanoparticle surface (appropriately determined based on the nanoparticle surface area), e.g., 10,000, 5,000, 2,500, 1,000, 750, 500, 250, or 200 oligonucleotides may be employed), with no limitations thereto. For instance, the number (m) of target-cloning strands per nanoparticle may range from 2 to 1,000, 2 to 500, 50 to 1,000, 50 to 500, 100 to 1,000, or 100 to 500, but is not limited thereto. In an embodiment, the number of cycles (n) may range from 1 to 20, 1 to 15, 1 to 10, 1 to 5, 2 to 20, 2 to 15, 2 to 10, 2 to 5, 3 to 20, 3 to 15, 3 to 10, or 3 to 5, with no limitations thereto.

In an embodiment of the composition, array, kit, device, and/or method for the amplification, detection, quantification, identification, and/or visualization of a target material, when the target material is a bioactive substance other than a nucleic acid molecule,
the substrate particles of (a) further include a capturing material for the target material in addition to the target-capturing strand previously described, and
the nanoparticles of (b) further include a binding material for the target material in addition to the partially double-stranded oligonucleotide previously described.

The binding material for the target material may be at least one selected from a group consisting of antibodies, aptamers, and small molecular chemicals that can bind (e.g., specifically bind) to a part of the target material. In this regard, the capturing material for the target material included in the substrate particles and the binding material for the target material included in the nanoparticles may bind to different parts of the target material. That is, the binding sites for the capturing material and the binding material on the target material may not overlap.

For the target material that is a bioactive substance other than a nucleic acid molecule, when the target material or sample comes into contact with the substrate particles to which the capturing material for the target material is immobilized, a part of the target material binds to the capturing material. Subsequent treatment with nanoparticles including the binding material for the target material leads to the formation of a complex between the substrate particles and nanoparticles through the binding of the binding material to the target material at a different site than the capturing material. Upon the formation of such complexes, dehybridizing the partially double-stranded oligonucleotide on the nanoparticles releases the target-cloning strand. Since the released target-cloning strand has the same nucleic acid sequence as the target oligonucleotide, it can hybridize with the target-capturing strand on the surface of the substrate particles, and the subsequent steps follow the same process as described earlier in (A) to (C), enabling the amplification and/or detection of bioactive substances other than nucleic acid molecules through the target-cloning strand. Thus, the presence and/or level of the bioactive substance in the sample can be measured through the amplified target-cloning strand and/or accumulated nanoparticles as described.

In the composition, array, kit, device, and/or method for the amplification, detection, quantification, identification, and/or visualization of target nucleic acid molecules or target materials, provided herein, the nanoparticles may be used in quantities significantly larger than the substrate particles to ensure enrichment on the surface of the substrate particles. Theoretically, the minimum number of nanoparticles per substrate particle may be appropriately selected from 2 to the number of target-capturing strands per substrate particle, but is not limited thereto. In an embodiment, the amount of nanoparticles and substrate particles used may be appropriately adjusted based on detection conditions, characteristics of the target sequence, etc. (for instance, if the target sequence has a strong hybridization (binding) nucleic acid sequence (e.g., "GC-rich" sequence), the amount of nanoparticles used may be relatively reduced, and if the sequence has a weak hybridization degree (e.g., "AT-rich" sequence), the amount of nanoparticles can be increased, but with no limitations thereto).

The composition, array, kit, device, and/or method for the amplification, detection, quantification, identification, and/or visualization of target nucleic acid molecules or target materials, provided herein, may be enzyme-free or polymerase-free.

Moreover, the method for the amplification, detection, quantification, identification, and/or visualization of target nucleic acid molecules or target materials, provided herein, may be performed in liquid phase.

In an embodiment, visualizable nanoparticles may be employed to intensify the detection/quantification/visualization by themselves, or alternatively, the target-cloning (target-amplification) strands can be labeled for detection/quantification/visualization. For instance, the target-cloning (target-amplification) strand may be labeled with a conventional marker. No particular limitations are imparted to markers that can be used, and any marker suitable for oligonucleotide labeling can be selected and used and may be, for example, fluorescent substances (such as fluorescent dyes and/or fluorescent proteins), but is not limited thereto.

The NCR (Nanoparticle Chain Reaction) technology provided herein can be applied to the multiplex detection and/or amplification of two or more different target materials by using two or more types of nanoparticles designed for different target materials (multiplexibility).

More specifically, the composition, array, kit, device, and/or method for the amplification, detection, quantification, identification, and/or visualization of target materials, provided herein, may utilize two or more types of nanoparticles that include template strands and target-cloning strands for different target materials (target nucleic acid molecules), and/or two or more types of substrate particles that include target-capturing strands for different target materials (target nucleic acid molecules), whereby the simultaneous amplification, detection, quantification, identification, and/or visualization of two or more target materials (target nucleic acid molecules) can be achieved. In the context of multiplex detection/amplification, if at least one of two or more types of the target materials is a substance other than a nucleic acid molecule, the nanoparticles may additionally include a binding material for the non-nucleic acid target material.

In multiplex detection/amplification, by labeling the target-cloning strands for each target material with different markers, two or more types of target materials can be amplified, detected, quantified, identified, and/or visualized simultaneously.

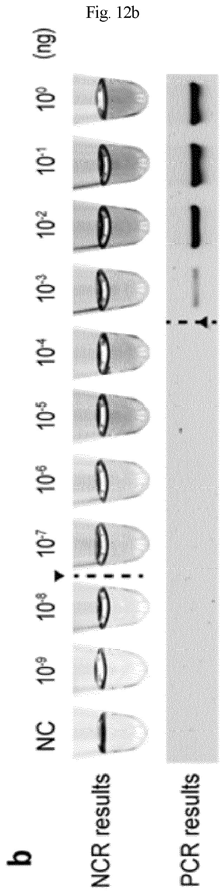

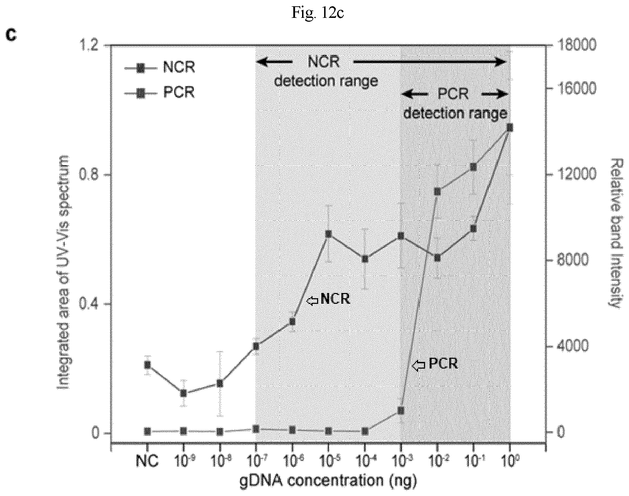

The composition, array, kit, device, and/or method provided herein can achieve significantly high detection sensitivity for target materials. For example, the detection sensitivity for target materials offered by the composition, array, kit, device, and/or method provided herein may be on the order of 10⁻⁷ng or higher, 10⁻⁶ng or higher, 10⁻⁵ng or higher, 10⁻⁴ng or higher, or 10⁻³ng or higher of the target material (for example, DNA (e.g., gDNA) or RNA) in the sample (that is, the lower limit of detectable amount of target material in the sample (detection limit) may be 10⁻⁷ng, 10⁻⁶ng, 10⁻⁵ng, 10⁻⁴ng, or 10⁻³ng). This represents a detection sensitivity significantly higher (for example, at least 10 times, at least 100 times, at least 1,000 times, or at least 10,000 times) than conventional PCR using polymerase (see FIG. 12b). In an embodiment, after 3 NCR cycles using NCR probes loaded with 174 target-cloning strands per nanoparticle, the dynamic range for target material (DNA) was found to range from about 100 zM to 100 pM as measured by solution color change or UV-Vis absorbance change (for example, measured at wavelengths of 400-650 nm) Moreover, the detection limit (LoD) identified by the integrated area of the UV-Vis spectrum was confirmed to be about 2 times, 10 times, 50 times, 100 times, 500 times, 1,000 times, 5,000 times, or 10,000 times higher in NCR compared to PCR (see FIG. 12c).

The NCR technology (composition, array, kit, device, and/or method) provided herein can be applied to quantitative diagnostic platforms. The composition, array, kit, device, and/or method provided herein can be applied to the amplification, detection, and quantification of various target materials, potentially replacing or augmenting traditional PCR technology, and can also be applied to diagnose diseases and/or symptoms related to the target material. Furthermore, the composition, array, kit, device, and/or method provided herein can be applied to rapid diagnostic technologies like Lateral Flow Assays (LFA), for example, in rapid diagnostic kits such as strip sensors, but are not limited thereto.

As previously described, developed according to the present application is a Nanoparticle Chain Reaction (NCR) technique that modifies PCR, which repeats enzyme-based target-amplifying thermal cycles, to detect target materials using visualizable nanoparticles (e.g., colorimetric detection). Specifically, when the target material is DNA and the nanoparticles are gold nanoparticles (AuNPs), the description will be explained with reference to the drawings, below:
Target DNA is amplified through a plurality of hybridized target-cloning DNA sequences released from nanoparticles (DNA-AuNPs) to which DNA with a partially double-stranded structure formed between a sequence identical to the target DNA (target-cloning DNA) and a complementary sequence binds. The amplification can be carried out by forming and separating target DNA sandwich complexes using DNA-modified magnetic particles, followed by releasing the target-cloning DNA strands along with the target DNA strands from the nanoparticles through a desalting process. The NCR technique provided herein can exponentially increase the target DNA by (the number of target-cloning DNAs per nanoparticle)ⁿ, where n is the number of cycles. Additionally, the NCR technique can dramatically increase the number of nanoparticles in the solution, allowing for the visual detection of a distinct solution color change induced by the plasmonic nanoparticles. NCR can repeat cycles until the accumulated amount of DNA-nanoparticles is sufficient for colorimetric detection based on eye vision or quantification based on UV-Vis spectrophotometry.

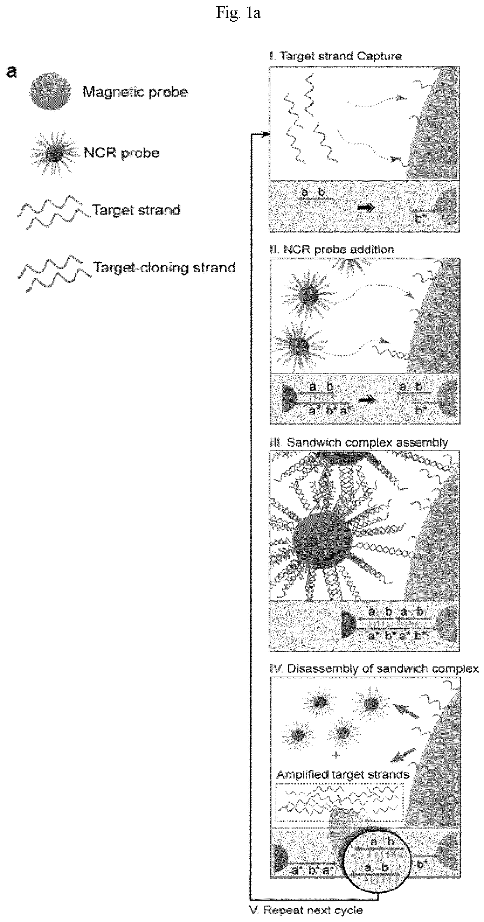

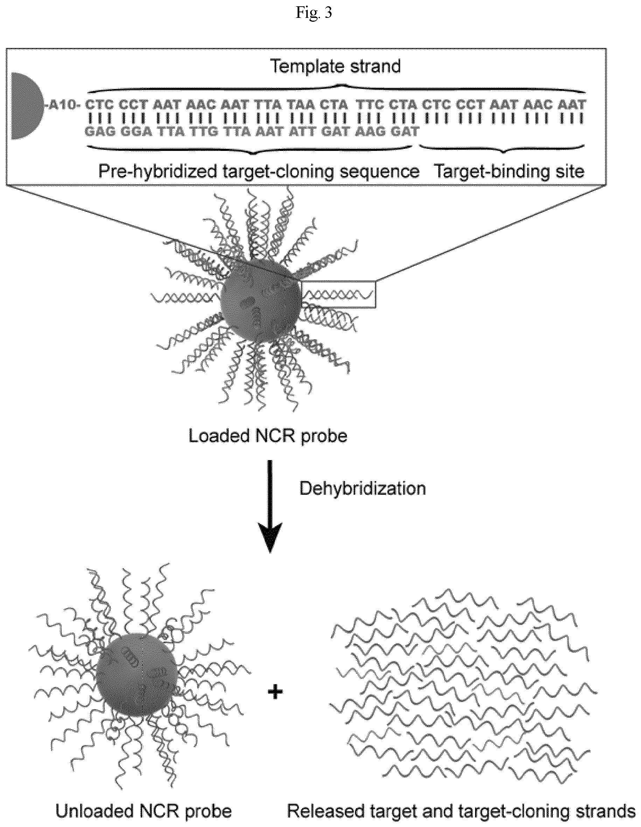

More specifically, after separation of the sandwich complexes by applying a magnetic field, the target-cloning DNA-loaded AuNP (NCR probe) is released from the complexes by dehybridization. The sequence of the target-cloning DNA is exactly identical to the target site sequence of the target DNA and is preloaded on the surface of the NCR probe before the assay. During this process, a plurality of target-cloning DNA strands are released along with the target strand. The released target-cloning strands are used in subsequent cycles for forming sandwich complexes of the NCR probe and magnetic particles (magnetic probe), thereby inducing a high level of opportunities for the formation of NCR probe-magnetic particle complexes (see FIG. 1a). Once the target-cloning strand-loaded NCR probe releases the target-cloning strand (dehybridization), the NCR probe becomes an unloaded NCR probe without the target-cloning strand (see FIG. 3). FIG. 3 schematically shows the release of the target-cloning strand from the loaded NCR probe, indicating that the loaded NCR probe includes pre-hybridized target-cloning strands (green) and template strands (red) with the target binding site, and upon dehybridization, the pre-hybridized target-cloning strand is released, turning the loaded NCR probe into an unloaded NCR probe for use in the next cycle of target amplification.

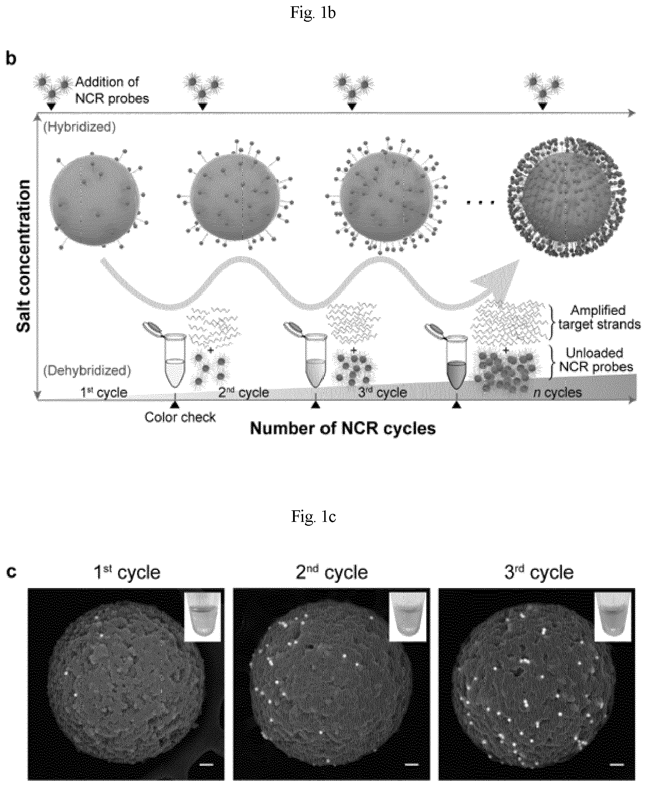

As the NCR cycles are repeated, the NCR probes accumulate to a high level, leading to an increase in the concentration of plasmonic AuNPs. This causes the solution to display a red color proportional to the concentration, and the total number of target strands and captured NCR probes in the reaction solution increases exponentially (FIG. 1b). The number of NCR probes captured on the surface of the magnetic particles increases with each NCR cycle, leading to an intensified red color of the solution, as shown by Scanning Electron Microscope (SEM) images of the NCR probe-magnetic particle sandwich complexes (FIGS. 1c, 2d, and 4).

To experimentally demonstrate the aforementioned content, the NCR technology provided herein was used to detect the DNA sequence of the anthrax lethal factor. Due to the importance of on-site diagnosis technologies that allow rapid and sensitive detection without special equipment, the gene related to anthrax was tested as a representative of infectious pathogens.

As illustrated in Example 1.3, NCR probes can be created by attaching thiolated template strands to gold nanoparticles (AuNPs) and then adding an excess of target-cloning strands to allow partial hybridization of the target-cloning strands to the template strands.

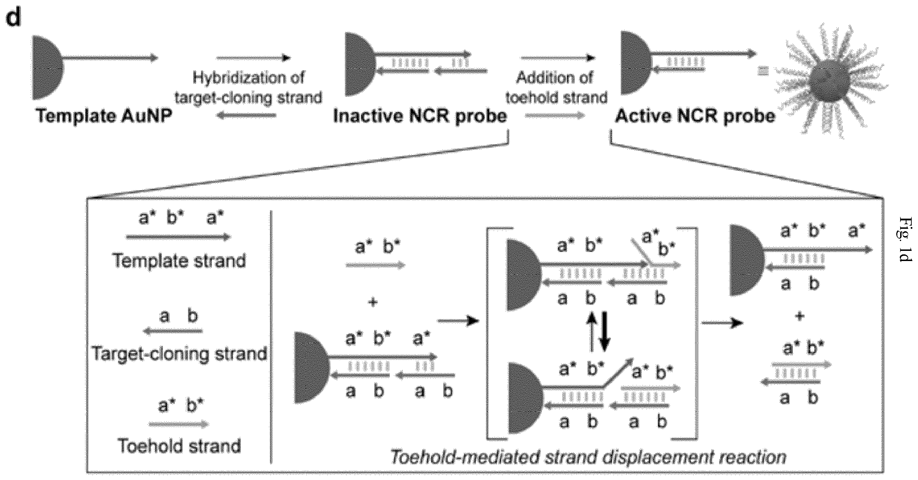

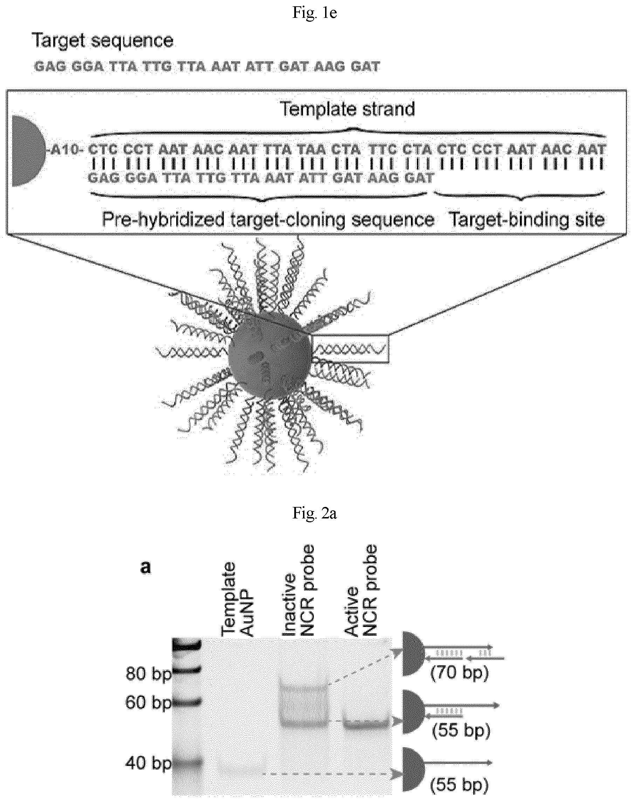

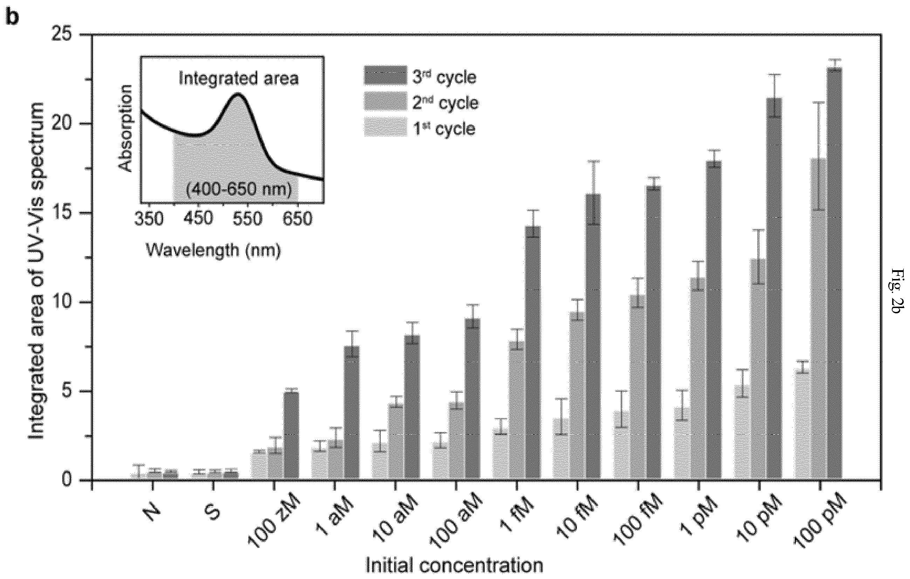

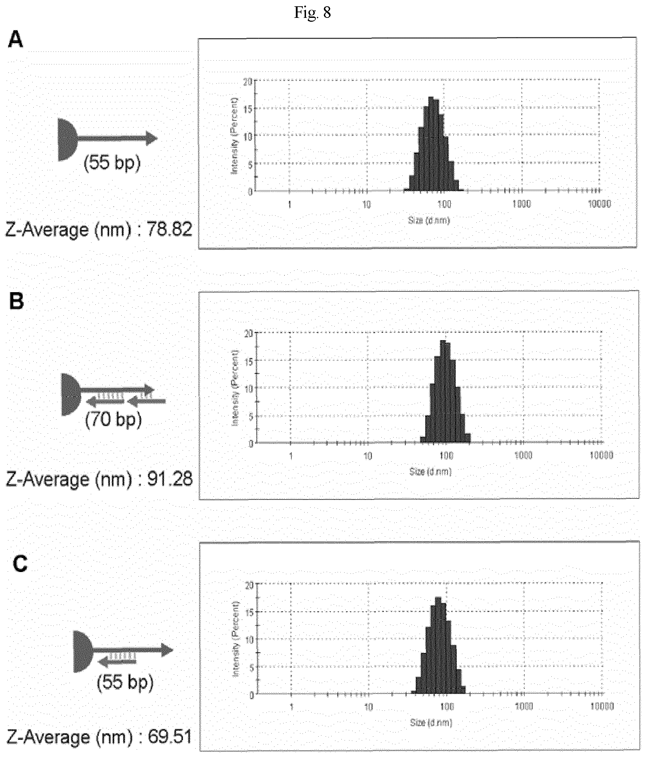

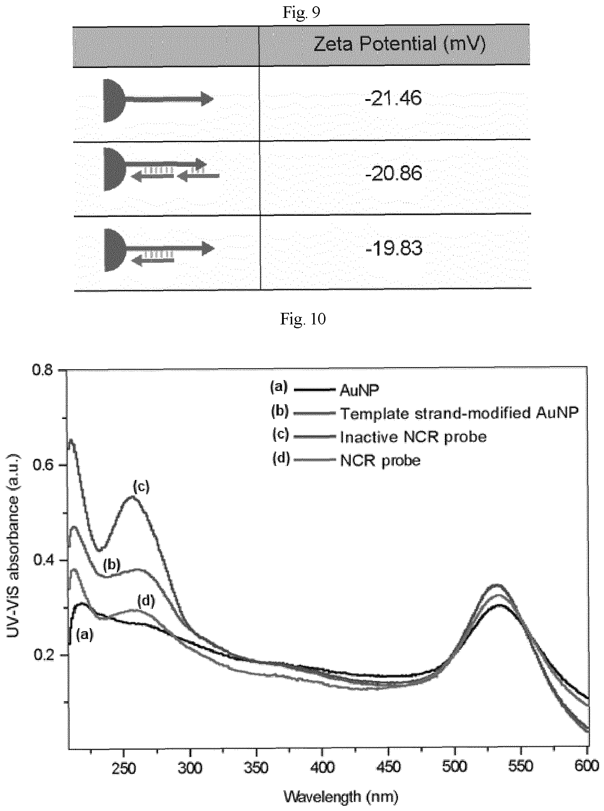

In this regard, since the target-binding site included within the template strand is complementary to a part of the target-cloning strand, undesired hybridization between the target-cloning strand and the target-binding site may occur, potentially leading to the formation of inactive NCR probes that fail to function properly and which severely degrade the selectivity and sensitivity of the NCR assay. To address this, adding target complements (toehold sequence) to induce toehold-mediated displacement can remove the undesirably hybridized target-cloning strands from the target-binding site of the template strand, thereby fully exposing the target-binding site for binding with the target strand and forming active NCR probes (see FIG. 1d). The results of gel electrophoresis for inactive NCR probes and NCR probes (with added toehold sequences) are shown in FIG. 2a. As illustrated in FIG. 2a, the intended toehold-mediated displacement exposing the target-binding site by adding the toehold sequence was confirmed. Furthermore, the dynamic light scattering (DLS) measurements of inactive and active NCR probes are presented in FIG. 8. As seen in FIG. 8, inactive NCR probes have a larger diameter than active NCR probes, with unloaded NCR probes showing the largest diameter. The zeta potential results are shown in FIG. 9, which demonstrates the surface negative charge state influenced by DNA conjugated onto the surface of NCR probes. FIG. 10 shows that AuNPs, template strand-modified (conjugated) AuNPs, inactive NCR probes, and active NCR probes generate diverse UV-Vis spectra. Notably, an absorption peak due to DNA at 260 nm and another absorption peak due to gold nanoparticles at 532 nm were observed. Importantly, after performing 2 cycles of NCR, the comparison between inactive and active NCR probes proved the enhanced selectivity of active NCR probes (see FIGS. 2b and 8). Active NCR probes showed a red color only in the presence of target NCR strands, whereas almost no detectable color was observed for non-target and single-base mismatch sequences. In contrast, inactive NCR probes displayed strong red coloration for target, non-target, and single-base mismatch sequences, indicating very low selectivity for the target sequence.

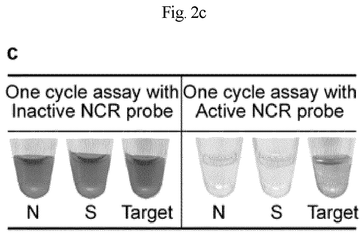

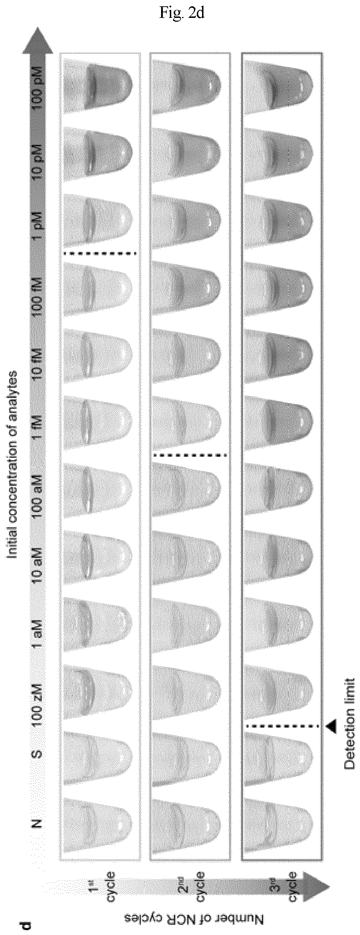

The increased number of captured NCR probes corresponds to the amplified amount of target nucleic acid with each repetition of the NCR cycle. Significantly, as the number of NCR cycles increases, there is an increase in the red intensity induced by AuNPs in the NCR assay products (see FIG. 2c). The visual detection limit of the NCR assay was confirmed to be at very low levels of approximately 1 pM, 1 fM, and 100 zM after 1, 2, and 3 NCR cycles, respectively. Overall, the red intensity of the NCR assay products gradually increased with the increase in target DNA concentration. For comparison, the detection of non-target and single-base mismatch sequences at 10 pM was conducted using the NCR assay, and in both cases, no detectable solution color change was observed. UV-Vis measurements were also performed to quantify the amount of target DNA more accurately through integrated AuNP-based spectral changes. To broadly cover the AuNP-induced spectral changes, the amount of AuNPs was obtained by integrating the UV-Vis spectral region at wavelengths from 400 to 650 nm. (FIG. 2d). Importantly, a more distinct increase in detection signal intensity was observed with the increase in target concentration as the number of NCR cycles increased, achieving a very wide dynamic range of 100zM to 100pM target concentration after 3 NCR cycles.

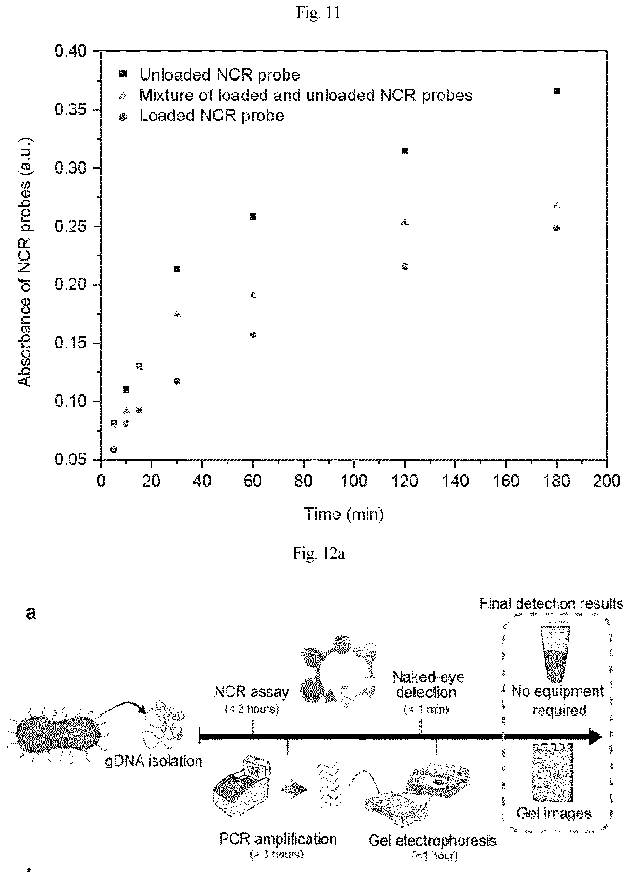

Then, the effect of increasing NCR cycles on the amount of target DNA was quantified by measuring the fluorescent signals using Cy3-labeled template strands and Cy5-labeled target-cloning strands. NCR probes were formed using these fluorophore-modified (labeled) strands, and AuNPs were finally dissolved with KCN to fully release the oligonucleotides for fluorescence-based quantification of DNA strands. FIG. 2e shows a much steeper increase in the number of target-cloning strands across a very wide dynamic range up to the fifth cycle of NCR. Nanoparticles provide a high surface-to-volume ratio, allowing a large number of nucleic acid molecules to be conjugated to the surface of AuNPs. In this embodiment, the average number of template strands is 430 strands per AuNP, and the number of pre-hybridized target-cloning strands is 174 strands per AuNP (FIGS. 9 and 10). Therefore, a single NCR probe can release up to 174 target-cloning strands per NCR cycle. In principle, while PCR amplifies the target strand by 2ⁿ times, the NCR provided herein can theoretically amplify the target strand by 174ⁿ times (where n represents the number of cycles), given the number of target-cloning strands bound to one nanoparticle is 174. However, it's important to note that the accumulated unloaded NCR probes could interfere with target-mediated hybridization between loaded NCR probes and DNA-conjugated magnetic particles. The analysis in the Examples indicated that unloaded NCR probes were more preferable for target binding than loaded NCR probes (see FIG. 11). Thus, the amplification efficiency of the NCR cycles has not been maximized in the current assay, and further optimization is needed. Nonetheless, the NCR assay enabled visual detection of zeptomolar target concentrations after just 3 NCR cycles.

### Advantageous Effects

Provided is a detection and/or diagnostic technique that allows for the stable amplification/detection/visualization of even minuscule amounts of target material using the novel nanoparticle chain reaction (NCR) technology. With no need of enzymes, the NCR technology requires no separate equipment while achieving very high detection sensitivity, and can be applied as an on-site detection/diagnostic tool. Compared to conventional PCR, the NCR technology can detect longer DNA (e.g., genomic DNA) with much higher sensitivity, thus improving the detection limit. Additionally, the NCR provided herein can contribute to an improvement in the detection limit of lateral flow assays (rapid kits) for on-site detection, finding advantageous applications in lateral flow assays (rapid kits).

### Description of Drawings

FIG. 1 illustrates a principle of the nanoparticle chain reaction through (a) one cycle step of nanoparticle chain reaction; (b) iterative NCR assay cycles; (c) SEM images of the sandwich complex of the NCR probe and magnetic substrate initiated by the 10 pM concentration of target analytes (scale bar, 200 nm; the inset shows naked eye detectable color appearance with increasing number of unloaded NCR probes); (d) a schematic diagram for synthetic preparation of NCR probe through the toehold-mediated strand displacement reaction; and (e) a schematic view of the NCR probe containing target-cloning strand (green color) and target binding site on the template strand (red color).
FIG. 2 shows colorimetric NCR assay results, wherein (a) gel electrophoresis was performed to show the desired NCR probe preparation process, with different DNA lengths obtained at each step of the NCR probe preparation; (b) the NCR results (UV-Vis spectra) for various concentrations of the target material are graphed, showing no detection of non-target sequence (N) and single-base mismatch sequence (S) probe and detection of the target material with high sensitivity (100 zM or higher), with detectability increasing with the number of cycles; and (c) and (d) NCR assay results can be detected with the naked eye.
FIG. 3 schematically illustrates release of target-cloning strands from loaded NCR probe.
FIG. 4 shows SEM images of sandwich complexes during the NCR assay, wherein the NCR assay was carried out with a 10 pM concentration of the target strand, with more sandwich complexes produced with more NCR cycles (scale bar: 1 µm).
FIG. 5 is a graph shows UV-Vis absorbance results after two NCR assay cycles.
FIG. 6 is a plot showing results of quantification of the target-cloning strands on the NCR probe.
FIG. 7 is a plot showing results of quantification of the template strands on the NCR probe.
FIG. 8 shows graphs of dynamic light scattering (DLS) measurements of NCR probes.
FIG. 9 shows results of zeta potential measurement of the NCR probes.
FIG. 10 is a plot showing UV-Vis spectrum measurement results of NCR probes.
FIG. 11 is a plot showing target strand-capturing capacity of loaded (target-cloning strand-hybridized partially double-stranded oligonucleotide included) and unloaded (only template strand included) NCR probes.
FIG. 12 shows comparisons between colorimetric NCR assay and PCR assay results, wherein (a) comparison of gDNA detection processes between NCR and PCR are schematically depicted; (b) gDNA extracted from bacteria are detected by NCR or PCR, illustrating the detection of NCR results with the naked eye (detection limit: 10⁻⁷ng) and PCR results via gel electrophoresis (detection limit: 10⁻³ng); and (c) NCR and PCR assay results (UV-Vis spectra) are plotted in comparison.

### Mode for Invention

A better understanding of the present disclosure may be obtained through the following examples which are set forth to illustrate, but are not to be construed to limit, the present disclosure.

### EXAMPLE 1

### 1.1. Material Preparation

Fifty-nm citrate-stabilized gold nanoparticles were purchased from BBI Solutions (Cardiff, UK). Dynabeads^{®} M-270 carboxylic acid was purchased from Thermo Fisher Scientific (Waltham, MA, USA). All chemicals were purchased from Sigma-Aldrich (St. Louis, MO, USA) and used as received without further purification. Oligonucleotides were obtained from Integrated DNA Technologies, Inc. Distilled water was obtained using a Millipore system (>18.0 MΩ, Milli-Q) and used in all the experiments. The Perfect Hyb^{™} Plus Hybridization buffer (Sigma-Aldrich) and 1x PBS buffer was mixed as ratio 1 to 3, and the mixed solution was used as hybridization buffer in NCR assay.

For the experimental validation of the effects of the present disclosure, the DNA sequence of anthrax lethal factor (GAG GGA TTA TTG TTA AAT ATT GAT AAG GAT; SEQ ID NO: 1) was selected as an oligonucleotide to be detected (hereinafter referred to as "target strand" or "target sequence") and used in subsequent experiments. The selection of the representative infectious pathogen anthrax-related gene as a detection target underscores the significance of developing rapid, sensitive, and equipment-free, on-site detection technologies.

### 1.2 Preparation of substrate particles (magnetic probes)

Carboxylic acid-functionalized magnetic particles (2.8 µm) were thoroughly vortexed for 10 minutes before use. Two hundreds µl of magnetic particles was transferred to a new tube. The magnetic particles were washed twice with 25 mM 2-[N-morpholino] ethane sulphonic acid (MES) buffer (pH 4.8) and resuspended in 20 µl of the same buffer. A mixture of 8 µl of N-ethyl-N'-[3-dimethylaminopropyl] carbodiimide hydrochloride (100 mg/ml) in 100 mM MES (pH 4.8) and 5'-amine-modified oligonucleotides (target-capturing strand) (10 nmol; 5'-NH₂-(CH₂)₃-A₁₀-ATC CTT ATC AAT ATT-3': NH₂-(CH₂)₃-A₁₀-[SEQ ID NO: 3])) was added to the washed magnetic-particle solution. After incubation for 4 hours at room temperature with vortexing, the magnetic substrates were washed three times and dispersed in hybridization buffer. The magnetic particles thus obtained are used as substrate particles for detecting a target material.

### 1.3. Preparation of NCR probes

The disulfide-modified template strand (5'-TAA CAA TAA TCC CTC ATC CTT ATC AAT ATT TAA CAA TAA TCC CTC (A)₁₀-3'; [SEQ ID NO: 2]-(A)₁₀) was reduced using 100 mM dithiothreitol (DTT) in a 100-mM phosphate buffer (pH 8) via incubation at room temperature for 2 hours to prepare a 3'-thiolated template strand (5'-[SEQ ID NO: 2]-PEG₆-A₁₀-SH-3'). Post reduction, excess DTT was removed using an NAP-5 column. The freshly reduced thiolated template strand was added in an amount of 10³ pmoles to 0.1%(v/v) SDS (sodium dodecyl sulfate) and 5.17 moles of gold nanoparticles (AuNPs) with an average diameter of 50 nm in distilled water. The mixed solution was incubated at room temperature for 2 hours and then adjusted to form a final phosphate concentration of 100 mM (pH 7.4). Six aliquots of 2 M NaCl with 0.01% SDS were added at 30-min intervals to obtain a final salt concentration of 0.3 M. The mixture was heated to 80°C for 10 min after each salt addition. The final mixture was incubated overnight by slowly cooling to room temperature to obtain a suspension of gold nanoparticles having a plurality of template strands bonded (conjugated) to the surface thereof. The suspension was washed via centrifugation (8000 rpm, 10 minutes), and the particle solution thus obtained was re-dispersed in distilled water. Template strand-modified gold nanoparticles were incubated with excess target-cloning strand (5'-GAG GGA TTA TTG TTA AAT ATT GAT AAG GAT-3'; SEQ ID NO: 1) in 300 mM NaCl buffer for 10 min at 55°C, and then cooled to room temperature for 1 hour. The excess target-cloning strand was removed by centrifugation (11,000 rpm, 2 minutes). Excess toehold DNA (SEQ ID NO: 4) in 40-mM MgCl₂ buffer with 0.1% SDS (vol/vol) was added to the same tube and incubated for 30 minutes at 43°C, followed by cooling down to room temperature for 3 hours to afford an NCR probe having the substrate strand bonded to the surface thereof, with the substrate strand partially hybridized with the target-cloning strand. After consecutive hybridizations, the NCR probe was washed four times by centrifugation (11000 rpm, 2 min) and re-dispersed in a hybridization buffer.

Sequences of the oligonucleotides used in Examples 1.2 and 1.3 are listed in Table 1, below:

**TABLE 1**

| DNA sequences used for NCR cycle | | |
|---|---|---|
| **Synthetic oligonucleotides** | **Sequences (5' → 3')** | **SEQ ID NO:** |
| Magnetic particle strand (target-capturing strand) | NH₂-(CH₂)₃-A₁₀-ATC CTT ATC AAT ATT | NH₂-(CH₂)₃-[SEQ ID NO: 3] |
| Template strand | TAA CAA TAA TCC CTC ATC CTT ATC AAT ATT TAA CAA TAA TCC CTC-PEG6- A10-SH | [SEQ ID NO: 2] - PEG₆-A₁₀-SH |
| Target-cloning strand | GAG GGA TTA TTG TTA AAT ATT GAT AAG GAT | SEQ ID NO: 1 |
| Toehold strand | ATC CTT ATC AAT ATT TAA CAA TAA TCC CTC | SEQ ID NO: 4 |
| Target strand | GAG GGA TTA TTG TTA AAT ATT GAT AAG GAT | SEQ ID NO: 1 |
| Single-base mismatch strand | GAG GGA TTA TTG TTA AAT ATT TAT AAG GAT | SEQ ID NO: 5 |
| Nonspecific strand | CTG ATT ACT ATT GCA TCT TCC GTT ACA ACT | SEQ ID NO: 6 |

### 1.4. NCR assay

Before amplification, Perfect Hyb^{™} Plus Hybridization buffer was mixed at a ratio of 1: 3 with 1x PBS buffer. For hybridization, 100 µl of the target strand (GAG GGA TTA TTG TTA AAT ATT GAT AAG GAT; SEQ ID NO: 1) and 10 µl of the magnetic substrate (Example 1.2, 5 mg/ml) were mixed and incubated for 10 minutes. To the mixture in the tube were added 15 µl of NCR probes before incubation for 20 min. Through a magnetic separator, the magnetic particles were washed three times with the hybridization buffer, dispersed in 30 ul of distilled water, and incubated at 80°C for 2 minutes for dehybridization. For the next cycle assay, a hybridization buffer was added to increase the salt concentration and then incubated for 10 minutes. Following addition of 15 µl of NCR (1OD)(5.17×10⁻² pmole/ml)(775amole), incubation was carried out for 20 minutes. The process of washing and release of nucleic acids was repeated, causing the assay products to show higher red color intensity

### 1.5. UV-Vis measurement of NCR assay product

The NCR assay products were isolated through the magnetic separator immediately and incubated at 80°C for nucleic acid release before UV-Vis measurement. The absorbance spectra of each released NCR assay product were measured using an ultraviolet-visible spectrometer (S-3100, SCINCO). The UV-Vis intensity of red color from gold nanoprobe was measured at 532 nm.

### 1.6. Polyacrylamide gel electrophoresis

To validate the active NCR probe preparation process including the toehold-mediated displacement reaction, polyacrylamide gel electrophoresis was performed. A 15% polyacrylamide gel was prepared using 30% acrylamide/Bis solution, deionized water, 5X TBE (Tris-Borate-EDTA) buffer, ammonium persulfate, and TEMED (tetramethylethylenediamine). The DNA-conjugated gold nanoparticles (Example 1.3) were treated with 0.1 M KCN solution to dissolve the gold nanoparticles. The gold-dissolved DNA samples with loading dye were added to each well of a 1.5-mm-thick gel. To confirm the DNA molecular weight on the gel, a 20-bp DNA ladder was loaded to another well. Using a power supply, an electric field was applied at 130 V for 70 min to the gel to conduct electrophoresis. After electrophoresis, the gel was stained using SYBR Green I (Lonza) for 10 min and imaged using UV light excitation at 306 nm by a gel imager.

### 1.7. Scanning electron microscopy (SEM)

The sandwich complex of magnetic substrate and NCR probes in the presence of target strands for each cycle were observed with FE-SEM (Field Emission Scanning Electron Microscope, SUPRA 55VP, Carl Zeiss, Germany). The sandwich complex structured particles were loaded on a copper grid, treated with Pt coating (EM ACE200, Leica, Austria) and imaged. FE-SEM imaging was carried out at the National Instrumentation Center for Environmental Management (Seoul National University, Seoul, South Korea).

### 1.8. Quantification of oligonucleotides

Fluorescent dye-modified oligonucleotides were used to prepare the AuNP-based NCR probes for quantification of the target oligonucleotides, and the fluorescence signals were measured using a fluorophotometer (F200 Pro, TECAN). The template strands and target-cloning strands were modified using Cy3 dye and Cy5 dye, respectively, at the 5' ends thereof. After salting and NCR assay, the gold nanoparticles were dissolved in 10 mM KCN. The remaining dye-modified oligonucleotides were used to measure the fluorescence intensity. The calibration curve of the known concentration of the dye sample was used for the quantification.

### 1.9. Dynamic light scattering (DLS) and Analysis of particle properties through zeta potential measurement

For the analysis of size and surface charge characteristics of NCR probes and magnetic substrate particles, the probes and/or particles were dispersed in distilled water solutions. The dispersion was measured for particle size and charge using a dynamic light scattering (DLS) photometer (Malvern Instrument, Malvern). The average values were calculated from three measurements.

### Data availability

The data that support the plots within this paper and other findings of this study are available from the corresponding author upon reasonable request.

### EXAMPLE 2. Colorimetric NCR Assay

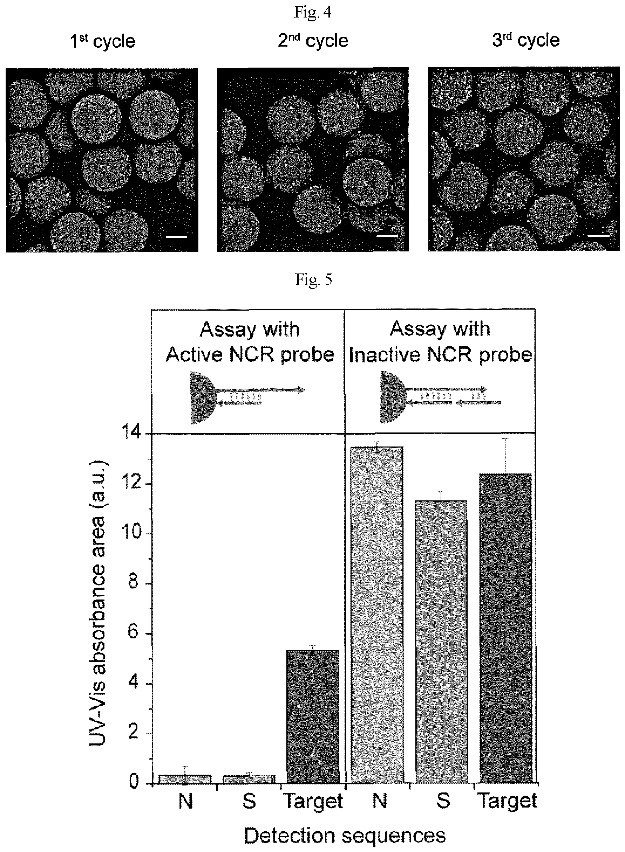

In Examples 1.2 and 1.3, magnetic probes and NCR probes fabricated respectively were treated with a 10 pM concentration of target oligonucleotide (GAG GGA TTA TTG TTA AAT ATT GAT AAG GAT; SEQ ID NO: 1). The sandwich complexes formed between the NCR probes and magnetic probes in each cycle were observed using Scanning Electron Microscopy (SEM) (see Example 1.7), and the color change in the reaction solution was visually observed. The obtained SEM images and solution color changes are shown in FIG. 1c (Scale bar, 200 nm; the inset shows the color appearance detectable by the naked eye as the number of unloaded NCR probes increases) and FIG. 4 (Scale bar: 1µm). As demonstrated by these results, with each repetition of the NCR cycle, the number of NCR probes captured on the surface of the magnetic probes increased, and the red intensity of the solution also increased.

In addition, to verify whether unintended binding between the template strand's target-binding site and the target-cloning strand occurred in the NCR probes prepared with the addition of toehold sequences (SEQ ID NO: 4), as in Example 1.3, gel electrophoresis was performed referring to Example 1.6. For comparison, the same test was performed on NCR probes that had not been treated with toehold DNA. The results of the obtained gel electrophoresis are shown in FIG. 2a. As depicted in FIG. 2a, in the case where the toehold sequence was added, as in Example 1.3, an active state of NCR probe was obtained where appropriate binding between the template strand's target-cloning site and the target-cloning strand occurs without binding between the template strand's target-binding site and the target-cloning strand, leaving the target-binding site exposed as a single strand (an overhang of the target-binding site). In contrast, in cases without the addition of toehold sequences, binding between the template strand's target-binding site and the target-cloning strand occurs, resulting in an inactive state of NCR probe with the target-binding site being not exposed as a single strand. These results indicate that the intended toehold-mediated displacement, which exposes the target-binding site by the addition of the toehold sequence, has occurred.

### EXAMPLE 3. NCR Assay (UV-Vis Spectrum)

NCR (nanoparticle chain reaction) assay results using various target oligonucleotide concentrations in the presence or absence of the toehold sequence were evaluated using UV-Vis spectra (see Examples 1.4 and 1.5).

To assess target specificity, the same tests were also conducted using a non-target sequence (N; SEQ ID NO: 6) and a single-base mismatch sequence (S; SEQ ID NO: 5) as comparison examples.

The results are depicted in FIGS. 2b to 2d.

FIG. 2b is a graph of the NCR results (UV-Vis spectrum) for various concentrations of the target sequence, exhibiting that while the non-target sequence (N) and the single-base mismatch sequence (S) are not detected, the target sequence is identified with high sensitivity (detection limit: 100 zM or higher), and the detection capability increases proportionally with the number of cycles. FIGS. 2c (after 1 cycle) and 2d (after 1 to 3 cycles) visually demonstrate the NCR assay outcomes, showing that the NCR assay does not detect non-target and single-base mismatch sequences, yet can efficiently detect the target sequence with high sensitivity. These findings emphasize that the NCR assay provided herein is target-specific and capable of amplifying/detecting/visualizing target materials with notable sensitivity. Moreover, FIG. 2c illustrates that the active NCR probe, with the template strand's target-binding site exposed, distinctly differentiates the target sequence from N and S sequences with the naked eye.

Additionally, after 2 cycles of the NCR assay, the UV-Vis absorbance range was measured and is depicted in FIG. 5. Here, the initial concentration of the target sequence was set to 10pM, and N and S represent the non-target sequence and the single-base mismatch sequence, respectively. The optical extinction values were measured at 532 nm (error bars represent the standard deviation of the extinction values from three independent experiments). As shown in FIG. 5, the active NCR probe, with the target-binding site of the template strand existing as an overhang, clearly allowed for visual distinction among the target sequence and N and S sequences by sight.

### EXAMPLE 4. Quantification of Oligonucleotides

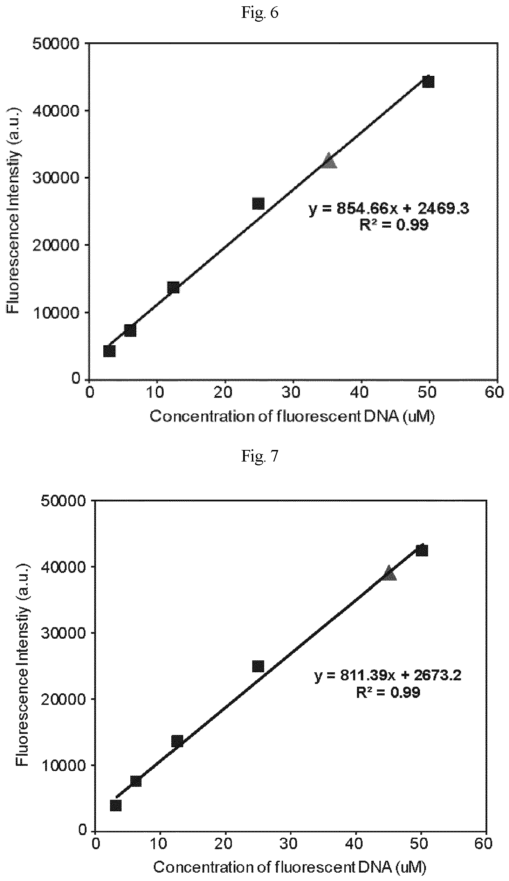

The quantification of oligonucleotides on NCR probes prepared in Example 1.3 was performed using the method described in Example 1.8, and the results are presented in FIGS. 6 and 7.

FIG. 6 illustrates the quantification results of the target-cloning strands on the NCR probes. The number of target-cloning strands on the AuNPs was calculated by measuring fluorescence intensity. NCR probes prepared with Cy5 dye-labeled target-cloning strands were used in the tests. A standard curve was constructed using known concentrations of Cy5 dye and serially diluted samples. The NCR probes (AuNP) were dissolved in 10 mM KCN before use, and the blue triangles on the standard curve represent the measured fluorescence intensity from the NCR probe solutions.

FIG. 7 displays the quantification results of the template strands on the NCR probes. The number of template strands per AuNP was calculated by measuring fluorescence intensity, using NCR probes obtained by conjugating Cy3 dye-labeled template strands to AuNPs. Standard curves were prepared with diluted samples and known concentrations of Cy3 dye. The NCR probes (AuNP) were also dissolved in 10 mM KCN for use. The red triangles on the standard curve indicate the fluorescence intensity measured from the template strand-bound AuNPs.

These results demonstrate the capability to accurately quantify oligonucleotides on the surface of nanoparticles, which is crucial for optimizing and understanding the efficiency of the NCR technology in target material amplification and detection.

### EXAMPLE 5. Measurement of Dynamic Light Scattering (DLS)

Dynamic light scattering (DLS) measurements were conducted on NCR probes as referenced in Example 1.9, and the results are depicted in FIGS. 8 to 11.

FIG. 8 displays the DLS measurement results for NCR probes. During the NCR probe fabrication process, the particle size changes were monitored by DLS. Gold nanoparticles modified (conjugated) with template strands showed a Z-average size of 78.82 nm. Inactive NCR probes, produced through target-cloning strand hybridization, exhibited a Z-average size of 91.28 nm, indicating longer DNA lengths compared to gold nanoparticles bound with template strands. Active NCR probes, after removing excess target-cloning strands via toehold sequence, were measured to have a reduced Z-average size of 69.51 nm, confirming a decrease in DNA length. Although the base numbers of template-modified AuNP and NCR probes were the same, the active NCR probes showed a smaller Z-average size because they include double-stranded DNA helix structures.

FIG. 9 presents the zeta potential measurement results for NCR probes. Surface charge changes during the NCR probe fabrication process can be measured as zeta potentials.

FIG. 10 shows UV-Vis spectrum measurement results for NCR probes. The UV-Vis absorbance spectra were measured at various stages of NCR probe fabrication. The data demonstrates a blue shift in UV-Vis data as DNA strands are conjugated to the surface of gold nanoparticles.

FIG. 11 is a plot showing the target strand capturing efficiency of loaded (including partially double-stranded oligonucleotides hybridized with target-cloning strands) and unloaded (containing only the template strand) NCR probes. Unloaded NCR probes are generated during the NCR assay and may interfere with the desired sandwich complex formation in the next cycle. To determine the impact of unloaded NCR probes during the NCR assay, the dynamics of unloaded and loaded NCR probes were measured through a single cycle NCR assay. For comparison, the same tests were performed on mixtures added to both probes at the same concentration, namely 10pM of target strands. Loaded and unloaded NCR probes were mixed at a ratio of 1:1 to prepare mixtures. It was observed that unloaded NCR probes demonstrated a more favorable reaction compared to loaded NCR probes.

### EXAMPLE 6. Comparison between Colorimetric NCR Assay and PCR

A comparison was conducted between the colorimetric NCR assay of this application and conventional PCR for detecting genomic DNA (gDNA) from bacteria *(Bacillus cereus).*

Since the bacteria share the sequence of SEQ ID NO: 1, which was used as a target in Examples 1.1 to 1.3, the NCR assay was carried out by the method of Example 1.4 using the magnetic probes and NCR probes prepared in Examples 1.1 to 1.3.

The PCR was performed using the following primer sequences and conditions:
F-primer : 5'-CTGGCTCAGGATGAACGC-3'(SEQ ID NO: 7)
R-primer : 5'-CGACTTCGGGTGTTACAA-3'(SEQ ID NO: 8)
PCR conditions: 35 cycles of 95°C for 1 minute, 53°C for 30 seconds, 72°C for 1 minute and 30 seconds.

The results comparing the colorimetric NCR assay and PCR are presented in FIGS. 12a to 12c. FIG. 12a is a schematic diagram comparing the gDNA detection process between NCR and PCR, highlighting that the NCR assay does not require equipment for detection and can be performed more quickly than PCR. FIG. 12b shows the results of detecting gDNA extracted from bacteria as analyzed by either the NCR or PCR methods, indicating that the NCR assay is naked-eye-detectable (detection limit: 10⁻⁷ng), whereas PCR requires gel electrophoresis for confirmation (detection limit: 10⁻³ng). FIG. 12c is a plot of the analysis results (UV-Vis spectrum) of NCR and PCR, demonstrating that the NCR assay exhibits approximately 10,000 times higher sensitivity than the PCR assay.

## Claims

1. A partially double-stranded oligonucleotide, comprising:
(1) a template strand comprising:
(i) a complementary target-cloning site to a target oligonucleotide, and
(ii) a complementary target-binding site to a portion of the target oligonucleotide; and
(2) a target-cloning strand comprising the same sequence as the target oligonucleotide, wherein the target-cloning strand hybridizes with the target-cloning site of the template strand to form a double-stranded structure and the target-binding site exists in a single-stranded form.

2. The partially double-stranded oligonucleotide of claim 1, wherein the template strand is 10-100 nt long.

3. A nanoparticle, comprising a plurality of partially double-stranded oligonucleotides, each comprising:
(1) a template strand comprising:
(i) a complementary target-cloning site to a target oligonucleotide, and
(ii) a complementary target-binding site to a portion of the target oligonucleotide; and
(2) a target-cloning strand comprising the same sequence as the target oligonucleotide, wherein the target-cloning strand hybridizes with the target-cloning site of the template strand to form a double-stranded structure and the target-binding site exists in a single-stranded form, and the one terminus of the template strand immobilized to a surface of the nanoparticle.

4. The nanoparticle of claim 3, wherein the nanoparticle is made of gold, silver, silica, platinum, copper, or an alloy of two or more selected from gold, silver, silica, platinum, and copper.

5. A composition for amplification, detection, quantification, or visualization of a target material, the composition comprising:
(a) a substrate particle having a plurality of target-capturing strands immobilized to a surface thereof, the target-capturing strand comprising a sequence complementary to a part (first part) of a target oligonucleotide, and
(b) a nanoparticle having a plurality of partially double-stranded oligonucleotides on a surface thereof, each of the partially double-stranded oligonucleotides comprising
(1) a template strand comprising
(i) a target-cloning site complementary to the target oligonucleotide, and
(ii) a target-binding site complementary to a part (second part) of the target oligonucleotide; and
(2) a target-cloning strand comprising the same sequence as the target oligonucleotide,
wherein the target-cloning site of the template strand complementarily hybridizes with the template strand to form a double-stranded structure, with an overhang of the target-binding site in the template strand,
a terminus of the template strand is immobilized to a surface of the nanoparticle, and the first part and the second part in the target oligonucleotide do not overlap.

6. The composition of claim 5, wherein the substrate particle and the nanoparticle each comprise two or more different types of particles and are used for amplifying, detecting, quantifying, or visualizing two or more types of target materials.

7. A method for amplification, detection, quantification, or visualization of a target material, the method comprising the steps of
(A) adding a target oligonucleotide or sample to a substrate particle having a plurality of target-capturing strands on a surface thereof to hybridize a part (first part) of the target oligonucleotide with the target-capturing strands;
(B) adding a plurality of nanoparticles to the mixture obtained in step (A) to form complexes of the substrate particles and nanoparticles, where each nanoparticle comprises a plurality of partially double-stranded oligonucleotides on a surface thereof,
each partially double-stranded oligonucleotide comprising
(1) a template strand comprising
(i) a target-cloning site complementary to the target oligonucleotide, and
(ii) a target-binding site complementary to a part (second part) of the target oligonucleotide; and
(2) a target-cloning strand comprising the same sequence as the target oligonucleotide,
wherein the target-cloning site of the template strand complementarily hybridizes with the target-cloning strand to form a double-stranded sequence, with the target-binding site remaining as a single strand, and
the complexes between the substrate particles and nanoparticles are formed through the hybridization of the part (second part) of the target oligonucleotide, which partially hybridizes with the target-capturing strands on the surface of the substrate particles, with the target-binding site; and
(C) performing dehybridization of the partially double-stranded oligonucleotide, disassembly of the complexes, or both thereof to release the target-cloning strand that has the same nucleic acid sequence as the target oligonucleotide.

8. The method of claim 7, wherein the target-cloning strand released in step (C) is used as a target oligonucleotide in step (A) to perform two or more cycles of steps (A) to (C).

9. The method of claim 7, wherein the substrate particle and the nanoparticle each comprise two or more different types of particles and are used for amplifying, detecting, quantifying, or visualizing two or more types of target materials.

10. The method of any one of claims 7 to 9, wherein the method is free of polymerase.

11. A kit for amplification, detection, quantification, or visualization of a target material, comprising the composition of claim 5 or 6.
